# EUROPEAN PATENT APPLICATION

(11) **EP 3 711 607 A1**
(43) Date of publication of application: **23.09.2020**
(21) Application number: 19164462.4
(22) Date of filing: 21.03.2019
(51) Int. Cl.: A24F 47/00, A61M 11/04, A61M 15/06

(54) **AEROSOL DELIVERY SYSTEM**

(71) Applicant: NERUDIA LIMITED, Liverpool Merseyside L24 9HP (GB)
(72) Inventor: The designation of the inventor has not yet been filed
(74) Representative: Mewburn Ellis LLP

(57) **Abstract**

An aerosol delivery system has a fluid-transfer article which holds an aerosol precursor and transfers that aerosol precursor to a transfer surface of the fluid-transfer article. The system comprises a heater, and the fluid-transfer article comprises a first region for holding an aerosol precursor and for transferring said aerosol precursor to the transfer surface. The heater comprises a porous element adjacent to but separable from said transfer surface of said fluid-transfer article and at least one heating element on an activation surface of said porous element, which activation surface is on the opposite side of the porous element from the fluid-transfer article.

## Description

### Field of the Invention

The present invention relates to an aerosol delivery system, and an aerosol-generation apparatus for an aerosol delivery system. The present invention preferably relates to an aerosol delivery system including a heater configured to heat an aerosol precursor to generate an aerosolised composition for inhalation by a user, and to an aerosol-generation apparatus therefor.

### Background

Pharmaceutical medicament, physiologically active substances and flavourings for example may be delivered to the human body by inhalation through the mouth and/or nose. Such material or substances may be delivered directly to the mucosa or mucous membrane lining the nasal and oral passages and/or the pulmonary system. For example, nicotine is consumed for therapeutic or recreational purposes and may be delivered to the body in a number of ways. Nicotine replacement therapies are aimed at people who wish to stop smoking and overcome their dependence on nicotine. Nicotine is delivered to the body in the form of aerosol delivery devices and systems, also known as smoking-substitute devices or nicotine delivery devices. Such devices may be non-powered or powered.

Devices or systems that are non-powered may comprise nicotine replacement therapy devices such as "inhalators", e.g. Nicorette® Inhalator. These generally have the appearance of a plastic cigarette and are used by people who crave the behaviour associated with consumption of combustible tobacco - the so-called hand-to-mouth aspect - of smoking tobacco. Inhalators generally allow nicotine-containing aerosol to be inhaled through an elongate tube in which a container containing a nicotine carrier, for example, a substrate, is located. An air stream caused by suction through the tube by the user carries nicotine vapours into the lungs of the user to satisfy a nicotine craving. The container may comprise a replaceable cartridge, which includes a cartridge housing and a passageway in the housing in which a nicotine reservoir is located. The reservoir holds a measured amount of nicotine in the form of the nicotine carrier. The measured amount of nicotine is an amount suitable for delivering a specific number of "doses". The form of the nicotine carrier is such as to allow nicotine vapour to be released into a fluid stream passing around or through the reservoir. This process is known as aerosolization and or atomization. Aerosolization is the process or act of converting a physical substance into the form of particles small and light enough to be carried on the air i.e. into an aerosol. Atomization is the process or act of separating or reducing a physical substance into fine particles and may include the generation of aerosols. The passageway generally has an opening at each end for communication with the exterior of the housing and for allowing the fluid stream through the passageway. A nicotine-impermeable barrier seals the reservoir from atmosphere. The barrier includes passageway barrier portions for sealing the passageway on both sides of the reservoir. These barrier portions are frangible so as to be penetrable for opening the passageway to atmosphere.

A device or a system that is powered can fall into two sub-categories. In both subcategories, such devices or systems may comprise electronic devices or systems that permit a user to simulate the act of smoking by producing an aerosol mist or vapour that is drawn into the lungs through the mouth and then exhaled. The electronic devices or systems typically cause the vaporization of a liquid containing nicotine and entrainment of the vapour into an airstream. Vaporization of an element or compound is a phase transition from the liquid phase to vapour i.e. evaporation or boiling. In use, the user experiences a similar satisfaction and physical sensation to those experienced from a traditional smoking or tobacco product, and exhales an aerosol mist or vapour of similar appearance to the smoke exhaled when using such traditional smoking or tobacco products.

A person of ordinary skill in the art will appreciate that devices or systems of the second, powered category as used herein include, but are not limited to, electronic nicotine delivery systems, electronic cigarettes, e-cigarettes, e-cigs, vaping cigarettes, pipes, cigars, cigarillos, vaporizers and devices of a similar nature that function to produce an aerosol mist or vapour that is inhaled by a user. Such nicotine delivery devices or systems of the second category incorporate a liquid reservoir element generally including a vaporizer or misting element such as a heating element or other suitable element, and are known, inter alia, as atomizers, cartomizers, or clearomizers. Some electronic cigarettes are disposable; others are reusable, with replaceable and refillable parts.

Aerosol delivery devices or systems in a first sub-category of the second, powered category generally use heat and/or ultrasonic agitation to vaporize a solution comprising nicotine and/or other flavouring, propylene glycol and/or glycerine-based base into an aerosol mist of vapour for inhalation.

Aerosol delivery devices or systems in a second sub-category of the second, powered category may typically comprise devices or systems in which tobacco is heated rather than combusted. During use, volatile compounds may be released from the tobacco by heat transfer from the heat source and entrained in air drawn through the aerosol delivery device or system. Direct contact between a heat source of the aerosol delivery device or system and the tobacco heats the tobacco to form an aerosol. As the aerosol containing the released compounds passes through the device, it cools and condenses to form an aerosol for inhalation by the user. In such devices or systems, heating, as opposed to burning, the tobacco may reduce the odour that can arise through combustion and pyrolytic degradation of tobacco.

Aerosol delivery devices or systems falling into the first sub-category of powered devices or systems may typically comprise a powered unit, comprising a heater element, which is arranged to heat a portion of a carrier that holds an aerosol precursor. The carrier comprises a substrate formed of a "wicking" material, which can absorb aerosol precursor liquid from a reservoir and hold the aerosol precursor liquid. Upon activation of the heater element, aerosol precursor liquid in the portion of the carrier in the vicinity of the heater element is vaporised and released from the carrier into an airstream flowing around the heater and carrier. Released aerosol precursor is entrained into the airstream to be borne by the airstream to an outlet of the device or system, from where it can be inhaled by a user.

The heater element is typically a resistive coil heater, which is wrapped around a portion of the carrier and is usually located in the liquid reservoir of the device or system. Consequently, the surface of the heater may always be in contact with the aerosol precursor liquid, and long-term exposure may result in the degradation of either or both of the liquid and heater. Furthermore, residues may build up upon the surface of the heater element, which may result in undesirable toxicants being inhaled by the user. Furthermore, as the level of liquid in the reservoir diminishes through use, regions of the heater element may become exposed and overheat.

The present invention has been devised in light of the above considerations.

### Summary of the Invention

At its most general, the present invention proposes that an aerosol generation apparatus has a fluid-transfer article which holds aerosol precursor and which transfers that aerosol precursor to a transfer surface. The fluid-transfer article is mounted adjacent (e.g. in contact with) a heater, so that the transfer surface is the closest part of the fluid-transfer article to the heater. The heater has a porous element, which allows aerosol precursor to pass from the transfer surface into the heater. The porous element has an activation surface on the opposite side of the porous element from the fluid-transfer article, on which activation surface is mounted at least one heating element for heating aerosol precursor which has reached the activation surface.

The separability of the fluid-transfer article and the heater means that the fluid-transfer article can be replaced without having to replace the heater. Since the aerosol precursor will be consumed when the apparatus is used by a user, it may be necessary to replace the fluid-transfer article, which acts as a reservoir for the aerosol precursor. The heater may remain and need not be replaced when the aerosol precursor is consumed.

The heater element or elements are normally mounted directly on the activation surface of the porous element of the heater. In such an arrangement, there will normally be an air-flow pathway adjacent the activation surface of the heater, so that vapour or aerosol/vapour mixture released from the activation surface by the heating effect of the heating element or elements may mix with the air-flow and pass to the user.

In such an arrangement, the airflow may have the effect of pulling liquid through the porous element from the fluid-transfer article onto the activation surface of the heater. This effect is assisted by the heating of the aerosol precursor by the heating element or elements, which causes the aerosol precursor to be liberated from the porous element as vapour or a vapour/aerosol mixture, thereby creating a flow of aerosol precursor through the porous heater.

Thus, according to the present invention, there may be provided an aerosol-generation apparatus comprising a heater and a fluid-transfer article, said fluid-transfer article comprising a first region for holding an aerosol precursor and for transferring said aerosol precursor to a transfer surface of said fluid-transfer article, the heater comprising a porous element adjacent to but separable from said transfer surface of said fluid-transfer article and at least one heating element on an activation surface of said porous element, which activation surface is on the opposite side of the porous element from the fluid-transfer article.

Preferably, said heating element or elements are mounted on said activation surface. The heating element or elements are then preferably coil, mesh or foil. There may be an air-flow pathway adjacent the activation surface.

The transfer surface of the fluid-transfer article may be planar, with the heater having a matching planar surface adjacent thereto. Thus, it is straightforward for aerosol precursor to pass from the transfer surface to the heater. Alternatively, the transfer surface and the adjacent surface of the heater may be convoluted, with a convolution to the transfer surface and the convolutions of the heater surface matching to provide mutual engagement. For example, the heater may have upwardly protruding triangular or conical projections that fit inside corresponding triangular or conical recesses in the transfer surface. Castellated and sinusoidal arrangements are also possible. Such convoluted arrangements have the advantage that they increase the surface area for liquid transfer between the transfer surface and the heater, although they require more manufacturing to achieve good mutual engagement.

The fluid-transfer article may act as a reservoir for aerosol precursor. Preferably, said first region of fluid-transfer article is of porous polymer material. The porous element of the heater may also be formed from porous polymer material. Alternatively, the porous element of the heater may be of fibrous material, such as ceramic fibre, glass fibre or carbon fibre, or from porous glass or porous ceramic.

The porous polymer material may comprise Polyetherimide (PEI) and/or Polyether ether ketone (PEEK) and/or Polytetrafluoroethylene (PTFE) and/or Polyimide (PI) and/or Polyethersulphone (PES) and/or Ultra-High Molecular Weight Polyethylene (UHMWPE) and/or Polypropylene (PP) and/or Polyethylene Terephthalate (PET).

The aerosol-generation apparatus may form part of an aerosol delivery system which has a carrier which includes a housing containing the fluid-transfer apparatus. There may then be a further housing containing the heater, with the housing and the further housing being separable. The further housing may have an inlet and outlet, with the air-flow pathway extending to the inlet and outlet.

The further housing may have a plate which is spaced from the activation of the porous structure of the heater, with the air-flow pathway passing between the activation surface and the plate.

The plate may have a plurality of recesses in its surface facing the activation surface, with the air-flow pathway passing through the recesses.

The invention includes the combination of the aspects and preferred features described except where such a combination is clearly impermissible or expressly avoided.

### Summary of the Figures

So that the invention may be understood, and so that further aspects and features thereof may be appreciated, embodiments illustrating the principles of the invention will now be discussed in further detail with reference to the accompanying figures, in which:
**Figure 1****.** is a perspective view illustration of a system for aerosol delivery according to one or more embodiments of the present invention;
**Figure 2****.** is a cross-section side view illustration of part of an apparatus of the system for aerosol delivery of Figure 1;
**Figure 3****.** is a cross-section side view illustration of the system and apparatus for aerosol delivery of Figure 1;
**Figure 4****.** is a perspective view illustration of an aerosol carrier for use in the system for aerosol delivery according to one or more embodiments of the present invention;
**Figure 5****.** is a cross-section side view of elements of an aerosol carrier and a part of an apparatus of the system for aerosol delivery according to one or more embodiments of the present invention;
**Figure 6****.** is a cross-section side view of elements of an aerosol carrier and a part of an apparatus of the system for aerosol delivery according to one or more embodiments of the present invention, in an alternative configuration from that of Figure 5;
**Figure 7****.** is a cross-section side view of elements of an aerosol carrier and a part of an apparatus of the system for aerosol delivery according to one or more embodiments of the present invention, in an alternative configuration from those of Figures 5 and 6;
**Figure 8****.** is a cross-section side view of aerosol carrier according to one or more embodiments of the present invention;
**Figure 9****.** is a perspective cross-section side view of the aerosol carrier of Figure 8, and;
**Figure 10****.** is an exploded perspective view illustration of a kit-of-parts for assembling the system according to one or more embodiments of the present invention.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

In general outline, one or more embodiments in accordance with the present invention may provide a system for aerosol delivery in which an aerosol carrier may be inserted into a receptacle (e.g. a "heating chamber") of an apparatus for initiating and maintaining release of an aerosol from the aerosol carrier. Another end, or another end portion, of the aerosol carrier may protrude from the apparatus and can be inserted into the mouth of a user for the inhalation of aerosol released from the aerosol carrier cartridge during operation of the apparatus.

Hereinafter, and for convenience only, "system for aerosol delivery" shall be referred to as "aerosol delivery system".

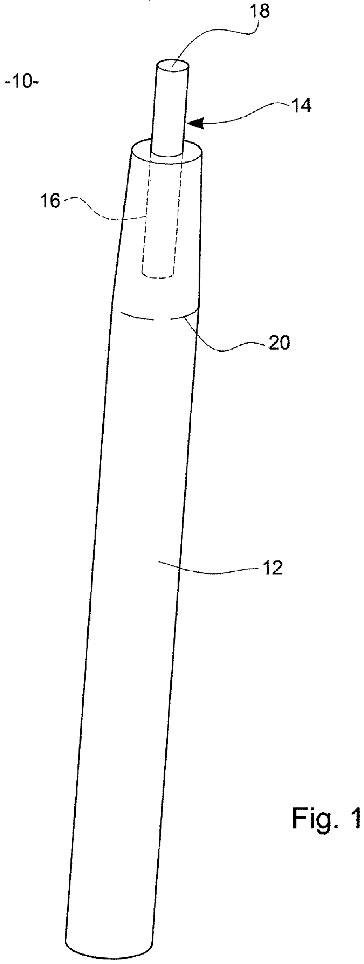

Referring now to Figure 1, there is illustrated a perspective view of an aerosol delivery system 10 comprising an aerosol generation apparatus 12 operative to initiate and maintain release of aerosol from a fluid-transfer article in an aerosol carrier 14. In the arrangement of Figure 1, the aerosol carrier 14 is shown with a first end 16 thereof and a portion of the length of the aerosol carrier 14 located within a receptacle of the apparatus 12. A remaining portion of the aerosol carrier 14 extends out of the receptacle. This remaining portion of the aerosol carrier 14, terminating at a second end 18 of the aerosol carrier, is configured for insertion into a user's mouth. A vapour and/or aerosol is produced when a heater (not shown in Figure 1) of the apparatus 12 heats a fluid-transfer article in the aerosol carrier 14 to release a vapour and/or an aerosol, and this can be delivered to the user, when the user sucks or inhales, via a fluid passage in communication with an outlet of the aerosol carrier 14 from the fluid-transfer article to the second end 18.

The device 12 also comprises air-intake apertures 20 in the housing of the apparatus 12 to provide a passage for air to be drawn into the interior of the apparatus 12 (when the user sucks or inhales) for delivery to a heater associated with the first end 16 of the aerosol carrier 14, so that the air can be drawn across an activation surface of the heater during use. Optionally, these apertures may be perforations in the housing of the apparatus 12.

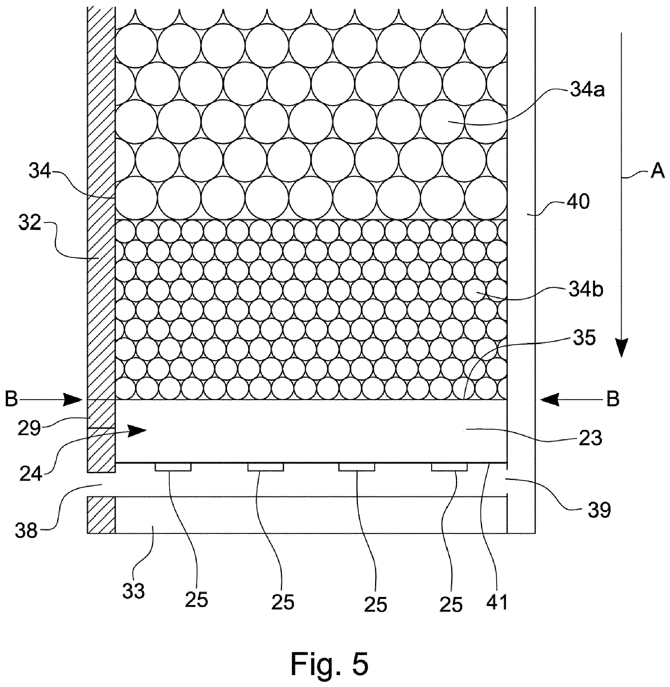

A fluid-transfer article (not shown in Figure 1, but described hereinafter with reference to Figs. 5 to 9 is located within a housing of the aerosol carrier 14. The fluid-transfer article contains an aerosol precursor material, which may include at least one of: nicotine; a nicotine precursor material; a nicotine compound; and one or more flavourings. As air passes across the activation surface of the heater, an aerosol may be entrained in the air stream, e.g. via diffusion to the air stream and/or via vaporisation of the aerosol precursor material and release from the heater under heating.

The substrate forming the fluid-transfer article 34 comprises a porous material where pores of the porous material hold, contain, carry, or bear the aerosol precursor material. In particular, the porous material of the fluid-transfer article is a porous polymer material such as, for example, a sintered material. Particular examples of material suitable for the fluid-transfer article include: Polyetherimide (PEI); Polytetrafluoroethylene (PTFE); Polyether ether ketone (PEEK); Polyimide (PI); Polyethersulphone (PES); and Ultra-High Molecular Weight Polyethylene. Other suitable materials may comprise, for example, BioVyonTM (by Porvair Filtration Group Ltd) and materials available from Porex®. Further optionally, a substrate forming the fluid-transfer article may comprise Polypropylene (PP) or Polyethylene Terephthalate (PET). All such materials may be described as heat resistant polymeric wicking material in the context of the present invention.

The aerosol carrier 14 is removable from the apparatus 12 so that it may be disposed of when expired. After removal of a used aerosol carrier 14, a replacement aerosol carrier 14 can be inserted into the apparatus 12 to replace the used aerosol carrier 14.

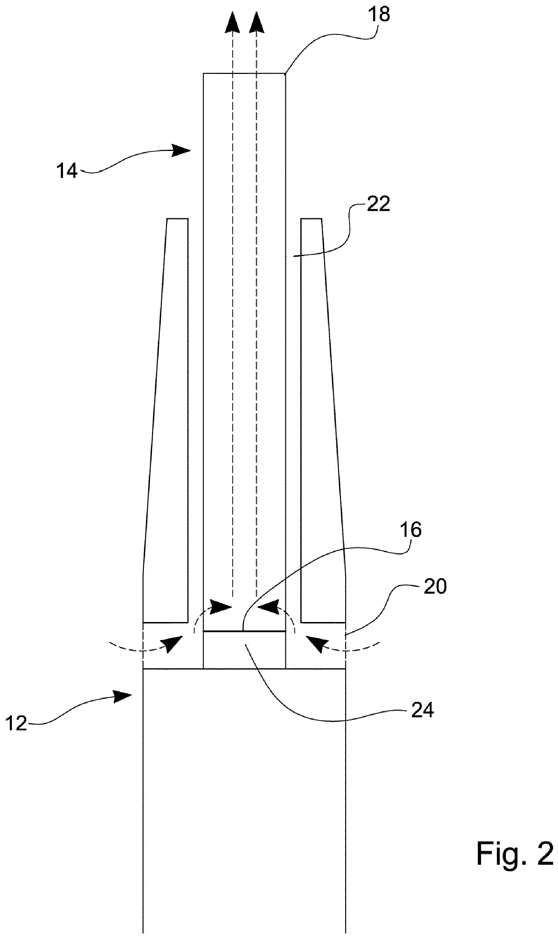

Figure 2 is a cross-sectional side view illustration of a part of apparatus 12 of the aerosol delivery system 10. The apparatus 12 comprises a receptacle 22 in which is located a portion of the aerosol carrier 14. In one or more optional arrangements, the receptacle 22 may enclose the aerosol carrier 14. The apparatus 12 also comprises a heater 24, which may contact a transfer surface of the fluid-transfer article (not shown in Figure 2) of the aerosol carrier 14 when an aerosol carrier 14 is located within the receptacle 22. Optional configurations of the heater 24 will be discussed later.

Air flows into the apparatus 12 (in particular, into a closed end of the receptacle 22) via air-intake apertures 20. From the closed end of the receptacle 22, the air is drawn into the aerosol carrier 14 (under the action of the user inhaling or sucking on the second end 18) and expelled at the second end 18. As the air flows towards the aerosol carrier 14, it passes across the activation surface of the heater. Heat from the heating elements of the heater 24 causes vaporisation of aerosol precursor material at the activation surface of the heater and an aerosol is created in the air flowing over the activation surface. Thus, through the application of heat in the region of the activation surface an aerosol is released, or liberated and is drawn from the material of the aerosol carrier unit by the air flowing across the activation surface and is transported in the air flow to via outlet conduits (not shown in Figure 2) in the housing of the aerosol carrier 14 to the second end 18. The direction of air flow is illustrated by arrows in Figure 2.

As a user sucks or inhales on second end 18 of the aerosol carrier 14, the is aerosol released from the heater and entrained in the air flowing across the activation surface of the heater is drawn through the outlet conduits (not shown) in the housing of the aerosol carrier 14 towards the second end 18 and onwards into the user's mouth.

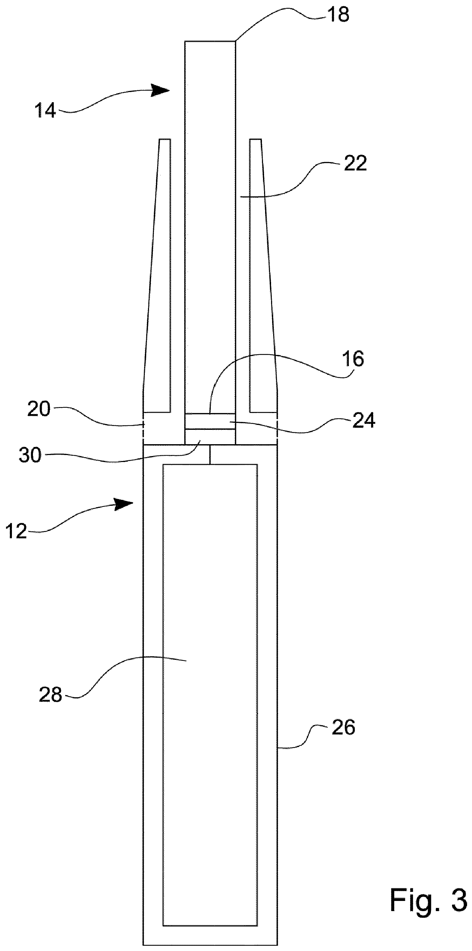

Turning now to Figure 3, a cross-sectional side view of the aerosol delivery system 10 is schematically illustrated showing the features described above in relation to Figures 1 and 2 in more detail. As can be seen, apparatus 12 comprises a housing 26, in which are located the receptacle 22 and heater 24. The housing 26 also contains control circuitry (not shown) operative by a user, or upon detection of air and/or vapour being drawn into the device 12 through air-intake apertures 20, i.e. when the user sucks or inhales. Additionally, the housing 26 comprises an electrical energy supply 28, for example a battery. Optionally, the battery comprises a rechargeable lithium ion battery. The housing 26 also comprises a coupling 30 for electrically (and optionally mechanically) coupling the electrical energy supply 28 to control circuitry (not shown) for powering and controlling operation of the heater 24.

Responsive to activation of the control circuitry of apparatus 12, the heating elements of the heater 24 cause a heating process to be initiated which causes (and, through continued operation, maintains) release of vapour and/or an aerosol from the activation surface of the heater. The vapour and/or aerosol formed as a result of the heating process is entrained into a stream of air being drawn across the activation surface of the heater (as the user sucks or inhales). The stream of air with the entrained vapour and/or aerosol passes through the aerosol carrier 14 via outlet conduits (not shown) and exits the aerosol carrier 14 at second end 18 for delivery to the user. This process is briefly described above in relation to Figure 2, where arrows schematically denote the flow of the air stream into the device 12 and through the aerosol carrier 14, and the flow of the air stream with the entrained vapour and/or aerosol through the aerosol carrier cartridge 14.

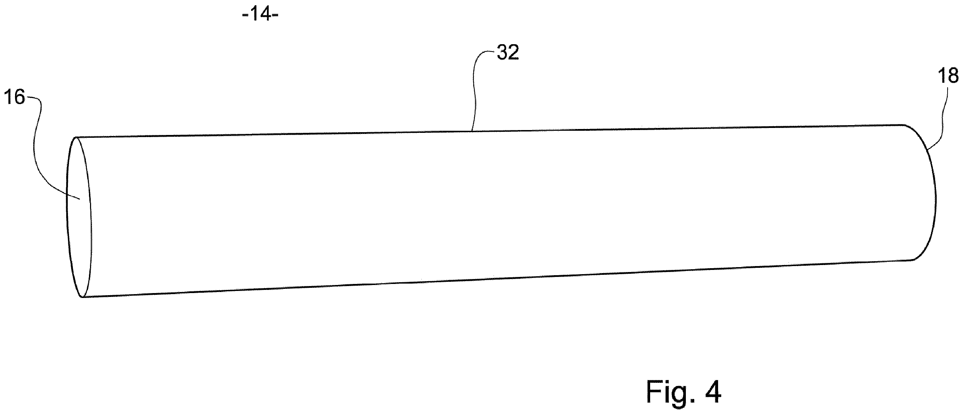

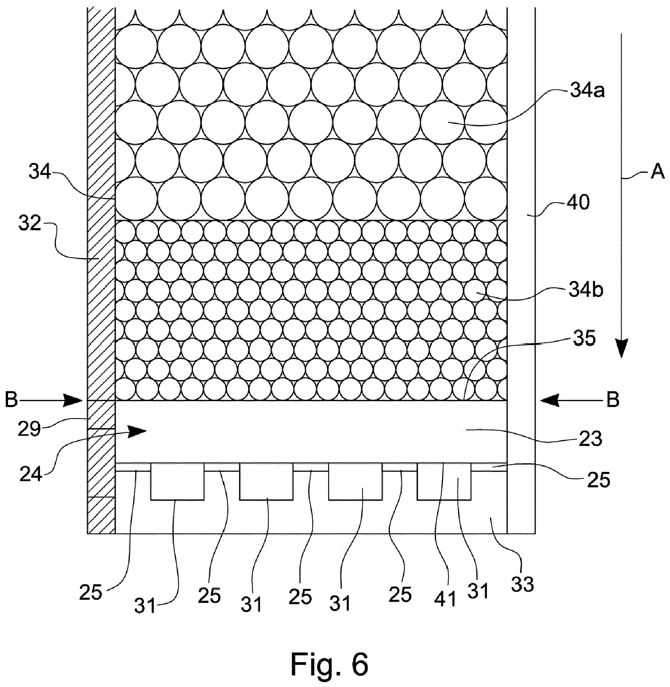

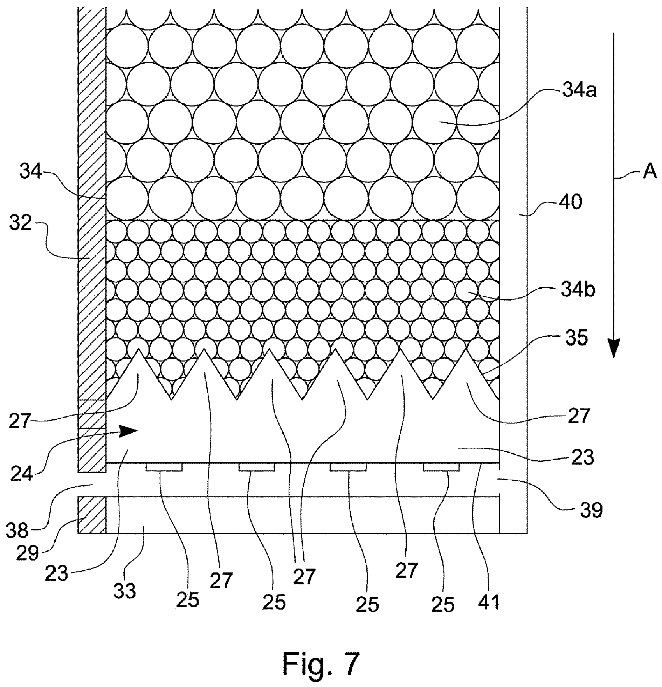

Figures 4 to 6 schematically illustrate the aerosol carrier 14 in more detail (and, in Figures 5, 6 and 7, features within the receptacle in more detail). Figure 4 illustrates an exterior of the aerosol carrier 14, Figure 5 illustrates internal components of the aerosol carrier 14 in one optional configuration, and Figs. 6 and 7 illustrate internal components of the aerosol carrier 14 in other optional configurations.

Fig. 4 illustrates the exterior of the aerosol carrier 14, which comprises housing 32 for housing said fluid-transfer article (not shown). The particular housing 32 illustrated in Figure 4 comprises a tubular member, which may be generally cylindrical in form, and which is configured to be received within the receptacle of the apparatus. First end 16 of the aerosol carrier 14 is for location to oppose the heater of the apparatus, and second end 18 (and the region adjacent the second end 18) is configured for insertion into a user's mouth.

Figure 5 illustrates some internal components of the aerosol carrier 14 and of the heater 24 of apparatus 12, in one embodiment of the invention.

As described above, the aerosol carrier 14 comprises a fluid-transfer element 34. The fluid-transfer article 34 may be removable from the housing 32, to enable it to be replaced. The fluid-transfer article 34 acts as a reservoir for aerosol precursor and that aerosol precursor will be consumed as the apparatus is used. Once sufficient aerosol precursor has been consumed, the aerosol precursor will need to be replaced. It may then be easiest to replace it by replacing the fluid-transfer article 34, rather than trying to re-fill the fluid-transfer article 34 with aerosol precursor while it is in the housing 32.

Adjacent to, but separable from, the fluid-transfer article 34 is the heater 24, which has an element 23 of a porous material which allows aerosol precursor to pass therethrough. In the arrangement of Figure 5 the porous element 23 of the heater 24 is in contact with transfer surface 35 of the fluid-transfer article 34, so that aerosol precursor may pass from that transfer surface 35 directly into the porous element 23 of the heater 24.

Since the element 23 is porous, the aerosol precursor will pass to the surface of the element 23 remote from the fluid-transfer article 34, which surface will be referred to as an activation surface 41. Heating elements 25 of the heater 24 are mounted on the activation surface 41. When the heating elements 25 are activated, the heat which they generate will be transferred to the activation surface 41.

Further components not shown in Figure 5 comprise: an inlet conduit, via which air can be drawn into the aerosol carrier 14; an outlet conduit, via which an air stream entrained with aerosol can be drawn from the aerosol carrier 14; a filter element; and a reservoir for storing aerosol precursor material and for providing the aerosol precursor material to the fluid-transfer article 34.

In Figure 5, the aerosol carrier is shown as comprising the fluid-transfer article 34 located within housing 32. The fluid transfer article 34 comprises a first region 34a holding an aerosol precursor. In one or more arrangements, the first region of 34a of the fluid transfer article 34 comprises a reservoir for holding the aerosol precursor. The first region 34a can be the sole reservoir of the aerosol carrier 14, or it can be arranged in fluid communication with a separate reservoir, where aerosol precursor is stored for supply to the first region 34a. The material forming the first region of 34a comprises a porous structure, whose pore diameter size may vary between one end of the first region 34a and another end of the first region 34a. For example, the pore diameter size may increase from a first end remote from heater 24 (the upper end is as shown in the figure) to a second end. The pore diameter size may change in a step-wise manner (i.e. a first part with pores having a diameter of first size, and a second part with pores having a diameter of second, smaller size), or the change in pore size in the first region 34a may be gradual rather than step-wise. This configuration of pores having a decreasing diameter size can provide a wicking effect, which can serve to draw fluid through the first region 34a, towards heater 24.

The fluid transfer article 34 also comprises a second region 34b. Aerosol precursor is drawn from the first region of 34a to the second region 34b by the wicking effect of the material forming the first region of 34a. Thus, the first region 34a is configured to transfer the aerosol precursor to the second region 34b of the article 34.

The second region 34b itself comprises a porous structure formed by a porous polymer material. It is then preferable that the pore diameter size of the porous structure of the second region 34b is smaller than the pore diameter size of the immediately adjacent part of the first region 34a. As mentioned above, the porous polymer material may be a sintered material. Particular examples of material suitable for the fluid-transfer article include: Polyetherimide (PEI); Polytetrafluoroethylene (PTFE); Polyether ether ketone (PEEK); Polyimide (PI); Polyethersulphone (PES); and Ultra-High Molecular Weight Polyethylene. Other suitable materials may comprise, for example, BioVyonTM (by Porvair Filtration Group Ltd) and materials available from Porex®. Further optionally, a substrate forming the fluid-transfer article may comprise Polypropylene (PP) or Polyethylene Terephthalate (PET).

As discussed above, the heating elements 25 transfer heat to the activation surface 41 of the heater, thereby releasing aerosol precursor which has reached that activation surface 41 through the porous polymer material of the second region 34b and the porous element 23 of the heater 24, in the form of vapour or a mixture of vapour and aerosol. That vapour and/or mixture of vapour and aerosol, may then pass into the air adjacent the activation surface 41 and the heating elements 25.

Figure 5 also illustrates an opening 38 in a further housing 29, which opening 38 is in communication with the air-intake apertures 20. A further opening 39 communicates with a duct 40, which duct 40 communicates with the second end 18. The housing 32 and the further housing 29 are separable, e.g. along the line B-B in Figure 5. This allows the housing 32 to be removed from the rest of the apparatus, when the aerosol precursor in the fluid-transfer article 34 has been consumed. The fluid-transfer article 34 can then be re-filled with aerosol precursor, on the fluid-transfer article 34 replaced by one filled with aerosol precursor. The further housing 29 may be integral with the housing 26 containing the electrical energy supply 28.

There is thus a fluid-flow path for air (hereinafter referred to as an air-flow pathway) between openings 38 and 39, linking the apertures 20 and the second end 18 of the aerosol carrier. When the user sucks or inhales, air is drawn along the air-flow pathway, along the activation surface 41. The housing 29 may include a plate 33 spaced from the activation surface 41, so that the air-flow pathway is defined between the activation surface 41 and the plate 33.

One or more droplets of the aerosol precursor will be released from the porous element 23 of the heater 24 and heated, to release vapour or a mixture of aerosol and vapour into the air flowing in the air-flow pathway between the openings 38, 39. The vapour or mixture passes, as the user sucks and inhales, to the second end 18.

The porous element 33 of the heater 24 may be fibrous, made from e.g. ceramic fibre, glass fibre or carbon fibre. Alternatively, it may be formed from a high-temperature porous material such as porous glass or porous ceramic.

It is thought that the flow of air between openings 38 and 39 along the activation surface 41 and past the heating elements 25 will have the effect of creating a lower air pressure adjacent the activation surface 41 which will tend to draw liquid through the porous element 23 to the activation surface 41. Thus, the transfer of aerosol precursor from the fluid-transfer article 34 is facilitated.

As mentioned above, the heater 24 is separable from the fluid-transfer article 34. The fluid-transfer article 34, formed by the first and second regions 34a and 34b and any further reservoir of aerosol precursor, may thus form a consumable part of the apparatus, in the sense that it can readily be replaced to enable the aerosol precursor to be replaced once it is consumed. The heater 24 formed by the porous element 23 and the heating elements 25 together with the surrounding housing 29 is not part of the consumable elements.

In Figure 5, the heating elements 25 may be separate or may be interconnected to form a single heating structure. For example, the heating elements 25 may be a coil, mesh or foil heater in which the heating elements 25 illustrated in Figure 5 are parts of a common structure. Such a coil, mesh or foil heater is preferred so that any restriction caused by the heating elements 25 on release of aerosol or vapour from the activation surface is minimised, as vapour and/or aerosol may pass through the heating elements 25. However, it is also possible for the heating elements 25 to be a solid unbroken strip or strips, provided that there is then enough of the activation surface 41 not covered by the heating elements 25 to allow sufficient release of vapour and/or aerosol from the activation surface 41.

In the illustrative examples of Figure 5, the first region 34a of the fluid-transfer article 34 is located at an "upstream" end of the fluid-transfer article 34 and the second region 34b is located at a downstream" end of the fluid-transfer article 34. That is, aerosol precursor is wicked, or is drawn, from the "upstream" end of the fluid-transfer article 34 to the "downstream" end of the fluid-transfer article 34 (as denoted by arrow A in Figure 5).

In the arrangement of Figure 5, the plate 33 has a planar surface facing the activation surface 41. Figure 6 illustrates an alternative arrangement in which the plate 33 has projections and recesses in its surface facing the activation surface 41, with the recesses forming channels 31 for air to flow therethrough. Thus, the channels 31 form the air-flow pathway along the activation surface 41. In Figure 6, the projections and recesses have a square-wave or "castellated" structure. Other shapes are possible, however, such as alternating peaks and troughs or recesses with curved or sinosoidal walls. All such arrangements permit channels 31 to be formed and allow air to flow along the activation surface 41. This control of air flow improves the mixing of the vapourised aerosol precursor into the air flow.

In the embodiment of Figure 6, the peaks in the upper surface of the plate 33 extend to the heating elements 25. Other alignments are possible, and the projections need not reach all the way to the heating elements 25. In general, however, the heating elements 25 may restrict release of the vaporised aerosol precursor from parts of the activation surface 41 on which those heating elements 25 are formed, so it will normally be desirable that the channels 31 are aligned with the part or parts of the activation surface 41 other than the part of parts on which the heating elements 25 are formed.

Note also that, in Figure 6, the openings 38 and 39 are not visible since they will be at the ends of the channels 31 to allow air to pass from the opening 38 in to the channels 31, and from those channels 31 out of the opening 39.

In the arrangements of Figures 5 and 6, the upper surface of the porous element 23 of the heater 24 which is adjacent the fluid-transfer article 34 is planar. Similarly, the lower surface of the fluid-transfer article 34, which forms the transfer surface 35, is also planar. Thus, the transfer surface 35 and the adjacent surface of the porous element 23 are in intimate contact, enabling good fluid transfer from the transfer surface to the pores of the porous element 23 of the heater 24 Such an arrangement is also simple to manufacture.

Figure 7 illustrates an embodiment corresponding to that illustrated in Figure 5, but in which the upper surface of the porous element 23 of the heater 24 comprises a plurality of V-shaped or triangular projections 27. Then, the transfer surface has matching V-shaped recesses in it, so that the transfer surface 35 follows the profiles of the projections 27. Thus, intimate contact between the transfer surface 35 and the heater is maintained, but the surface area of contact is increased, thereby promoting transfer of aerosol precursor from the fluid-transfer article 32 to the porous element 23 of the heater 24. Other possible configurations for the interface between the fluid-transfer article 32 and the heater 24 can be used, such as "castellated" or "sinusoidal" arrangements. It is then a balance between the increased complexity of manufacture to provide such convoluted surfaces, and the increased fluid transfer which results.

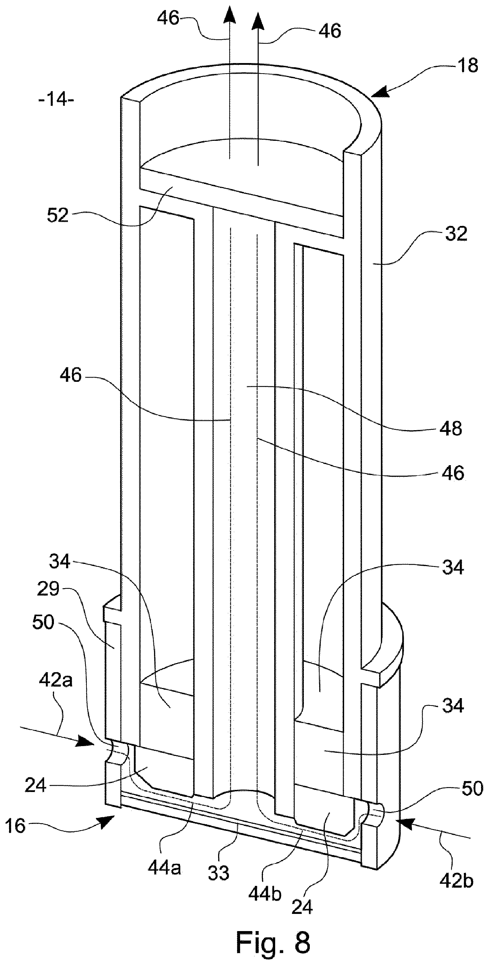

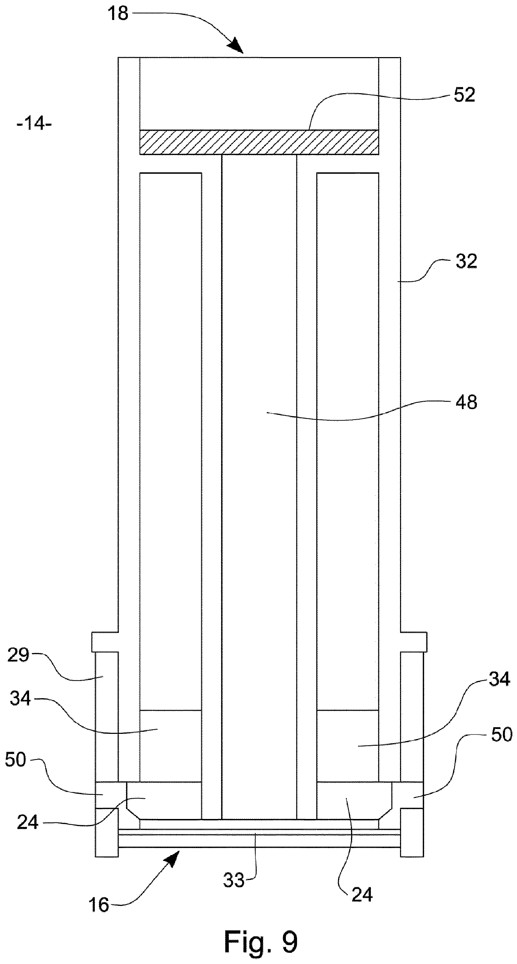

In the arrangements shown in Figures 5 to 7, the apertures 38, 39 are on opposite sides of the housing 32. Figures 8 and 9 show an alternative configuration, in which the fluid-transfer article is annular, and both the fluid-transfer article 34 and the intermediate structure 36 is then in the form of annulus. In Figures 8 and 9, the structure of the fluid-transfer article 34 and the intermediate structure correspond to that shown in Figure 5. The internal structure of fluid-transfer article 34 and heater 24 may be the same as in Figures 5 to 7, but is not illustrated in detail in Figures 8 and 9 for simplicity. The heating elements 25 also cannot be seen in Figures 8 and 9, but may be formed as in the arrangement of Figure 5 or Figure 6. However, the air flow in the apparatus is discussed in more detail below. Thus, Figures 8 and 9 illustrate an aerosol carrier 14 according to one or more possible arrangements in more detail. Figure 7 is a cross-section side view illustration of the aerosol carrier 14 and Figure 8 is a perspective cross-section side view illustration of the aerosol carrier 14.

As can be seen from Figures 8 and 9, the aerosol carrier 14 is generally tubular in form. The aerosol carrier 14 comprises housing 32, which defines the external walls of the aerosol carrier 14 and which defines therein a chamber in which are disposed the fluid-transfer article 34 (adjacent the first end 16 of the aerosol carrier 14) and internal walls defining the fluid communication pathway 48. Fluid communication pathway 48 defines a fluid pathway for an outgoing air stream from the channels 40 to the second end 18 of the aerosol carrier 14. In the examples illustrated in Figures 8 and 9, the fluid-transfer article 34 is an annular shaped element located around the fluid communication pathway 48.

In walls of the housing 29, there are provided inlet apertures 50 to provide a fluid communication pathway for an incoming air stream to reach the activation surface 41 of the heater 24. As is in the arrangements of Figures 5 to 7, the housings 29 and 32 are separable in Figures 8 and 9.

In the illustrated example of Figures 8 and 9, the aerosol carrier 14 further comprises a filter element 52. The filter element 52 is located across the fluid communication pathway 48 such that an outgoing air stream passing through the fluid communication pathway 48 passes through the filter element 52.

With reference to Figure 8, when a user sucks on a mouthpiece of the apparatus (or on the second end 18 of the aerosol carrier 14, if configured as a mouthpiece), air is drawn into the carrier through inlet apertures 50 extending through walls in the housing 29 of the aerosol carrier 14.

An incoming air stream 42a from a first side of the aerosol carrier 14 is directed to a first side of the heater 24 (e.g. via a gas communication pathway within the housing of the carrier). An incoming air stream 42b from a second side of the aerosol carrier 14 is directed to a second side of the heater 24(e.g. via a gas communication pathway within the housing of the carrier). When the incoming air stream 42a from the first side of the aerosol carrier 14 reaches the first side of the heater 24, the incoming air stream 42a from the first side of the aerosol carrier 14 flows along the activation surface of the heater 24. Likewise, when the incoming air stream 42b from the second side of the aerosol carrier 14 reaches the second side of the heater 24, the incoming air stream 42b from the second side of the aerosol carrier 14 flows along the activation surface of the heater 24. The air streams from each side are denoted by dashed lines 44a and 44b in Figure 8. As these air streams 44a and 44b flow, aerosol precursor on the activation surface 41 of the heater 24 is entrained in air streams 44a and 44b.

In use, the heating elements 25 of the apparatus 12 raise the temperature of the porous element 23 of the heater 24 to a sufficient temperature to release, or liberate, captive substances (i.e. the aerosol precursor) to form a vapour and/or aerosol, which is drawn downstream. As the air streams 44a and 44b continue their passages, more released aerosol precursor is entrained within the air streams 44a and 44b. When the air streams 44a and 44b entrained with aerosol precursor meet at a mouth of the outlet fluid communication pathway 48, they enter the outlet fluid communication pathway 48 and continue until they pass through filter element 52 and exit outlet fluid communication pathway 48, either as a single outgoing air stream, or as separate outgoing air streams 46 (as shown). The outgoing air streams 46 are directed to an outlet, from where it can be inhaled by the user directly (if the second end 18 of the aerosol capsule 14 is configured as a mouthpiece), or via a mouthpiece. The outgoing air streams 46 entrained with aerosol precursor are directed to the outlet (e.g. via a gas communication pathway within the housing of the carrier).

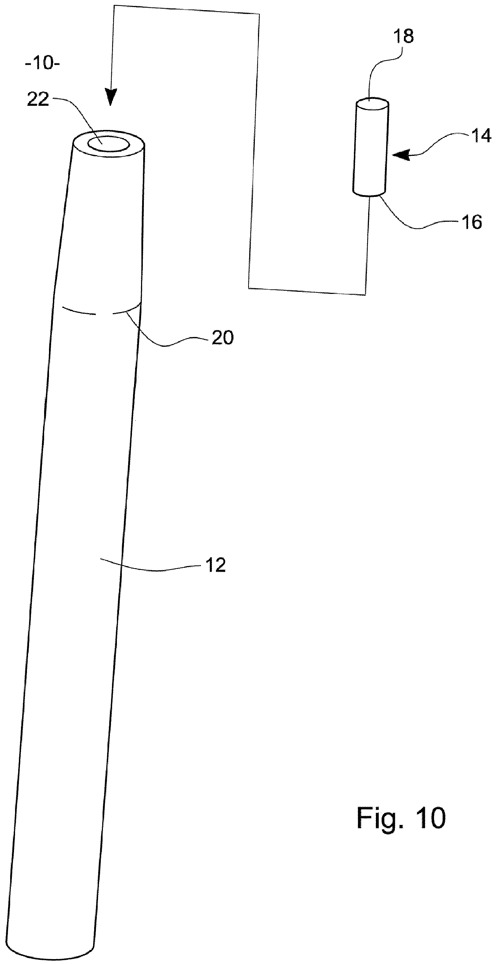

Figure 10 is an exploded perspective view illustration of a kit-of-parts for assembling an aerosol delivery system 10.

As will be appreciated, in the arrangements described above, the fluid-transfer article 34 is provided within a housing 32 of the aerosol carrier 14. In such arrangements, the housing of the carrier 14 serves to protect the aerosol precursor-containing fluid-transfer article 34, whilst also allowing the carrier 14 to be handled by a user without his/her fingers coming into contact with the aerosol precursor liquid retained therein.

In any of the embodiments described above the second region 34b may have a thickness of less than 5mm. In other embodiments it may have a thickness of: less than 3.5mm, less than 3mm, less than 2.5mm, less than 2mm, less than 1.9mm, less than 1.8mm, less than 1.7mm, less than 1.6mm, less than 1.5mm, less than 1.4mm, less than 1.3mm, less than 1.2mm, less than 1.1mm, less than 1mm, less than 0.9mm, less than 0.8mm, less than 0.7mm, less than 0.6mm, less than 0.5mm, less than 0.4mm, less than 0.3mm, less than 0.2mm, or less than 0.1mm.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. An aerosol-generation apparatus comprising a heater and a fluid-transfer article, said fluid-transfer article comprising a first region for holding an aerosol precursor and for transferring said aerosol precursor to a transfer surface of said fluid-transfer article, the heater comprising a porous element adjacent to but separable from said transfer surface of said fluid-transfer article and at least one heating element on an activation surface of said porous element, which activation surface is on the opposite side of the porous element from the fluid-transfer article.

2. An aerosol-generation apparatus according to claim 2, wherein said at least one heating element is a coil, mesh or foil.

3. An aerosol-generation apparatus according to claim 1, or claim 2, having an air-flow pathway adjacent said activation surface.

4. An aerosol-generation apparatus according to any of the claims 1 to 3, wherein said transfer surface is planar, and the surface of said heater adjacent said transfer surface is also planar.

5. An aerosol-generation apparatus according to any of the claims 1 to 3, wherein said transfer surface is convoluted, and the surface of said heater adjacent said transfer surface is also convoluted, the convolutions of the transfer surface and the convolutions of the heater surface matching to provide mutual engagement.

6. An aerosol-generation apparatus according to any one of the preceding claims, wherein said porous element is formed of a polymer material.

7. An aerosol-generation apparatus according to any one of the preceding claims, wherein said porous element is formed of fibrous material.

8. An aerosol precursor according to claim 7, wherein said fibrous material is ceramic fibre, glass fibre or carbon fibre.

9. An aerosol-generation apparatus according to any one of the preceding claims, wherein said fluid-transfer article has a second region of porous polymer material, said transfer surface being a surface of said second region, and said first region of said fluid-transfer article being arranged to transfer said aerosol precursor to said second region.

10. An aerosol-generation apparatus according to any of the preceding claims, wherein the first region of said fluid-transfer article is formed from porous polymer material.

11. An aerosol delivery system comprising an aerosol-generation apparatus according to any one of the claims 1 to 10 and a carrier comprising a housing containing said fluid-transfer article.

12. An aerosol-delivery system according to claim 11, having a further housing containing said heater, said housing and said further housing being separable.

13. An aerosol delivery system according to claim 12 as dependent on claim 3, wherein said further housing has an inlet and an outlet and said air-flow pathway extends to said inlet and said outlet.

14. An aerosol delivery system according to claim 13, wherein said further housing has a plate and said heater is mounted in the further housing so that the activation surface is spaced from said plate and said air-flow pathway passes between said activation surface and said plate.

15. An aerosol delivery system according to claim 14, wherein said plate has a plurality of recesses in its surface facing the activation surface, the air-flow pathway passing through said recesses.
